## Europäisches Patentamt
## European Patent Office
## Office européen des brevets

(19)

(11) Veröffentlichungsnummer: **0 001 289**
**B1**

(12) **EUROPÄISCHE PATENTSCHRIFT**

(45) Veröffentlichungstag der Patentschrift: **14.10.81**

(51) Int. Cl.³: **A 61 B 17/18, A 61 N 1/40**

(21) Anmeldenummer: **78100966.7**

(22) Anmeldetag: **22.09.78**

(54) **Zusatzvorrichtung zum Umwandeln eines konventionellen Osteosyntheseimplantats in ein Elektro-Osteosynthese-implantat.**

(30) Priorität: **22.09.77 DE 2742741**
**12.06.78 DE 2825719**

(73) Patentinhaber: **Kraus, Werner**
**Augustenstrasse 41**
**D-8000 München 2 (DE)**

(43) Veröffentlichungstag der Anmeldung:
**04.04.79 Patentblatt 79/7**

(72) Erfinder: **Kraus, Werner**
**Augustenstrasse 41**
**D-8000 München 2 (DE)**

(45) Bekanntmachung des Hinweises auf die Patenterteilung:
**14.10.81 Patentblatt 81/41**

(74) Vertreter: **von Bezold, Dieter, Dr. et al,**
**Postfach 86 02 60**
**D-8000 München 86 (DE)**

(84) Benannte Vertragsstaaten:
**BE CH DE FR GB NL SE**

(56) Entgegenhaltungen:
**DE - A - 2 617 240**
**DE - A - 2 636 818**

Courier Press, Leamington Spa, England.

## Zusatzvorrichtung zum Umwandeln eines konventionellen Osteosyntheseimplantats in ein Electro-Osteosyntheseimplantat

Die vorliegende Erfindung betrifft eine Zusatzvorrichtung zum Umwandeln eines konventionellen Osteosytheseimplantats in Form einer Osteosyntheseplatte oder eines Marknagels in ein entsprechendes Elektro-Osteosyntheseimplantat.

Aus der DE—A—1 918 299 (US—A—3 745 995) und der CH—A—551 201 (US—A— 3 820 534) sind Osteosyntheseimplantate bekannt, in die eine gegebenenfalls mit einem Magnetkern versehene induktive Aufnehmerspule eingebaut ist, die zwei oder mehr Wicklungsanschlüsse aufweist. Die Wicklungsanschlüsse sind mit Elektroden verbunden, welche im beschädigten Bereich des durch das Osteosyntheseimplantat mechanisch fixierten Knochens angeordnet werden. Nach dem Verschließen des Wundbereiches wird in der Aufnehmerspule ein niederfrequenter elektrischer Wechselstrom induziert, der über die Elektroden durch den beschädigten Knochenbereich fließt und eine Neubildung von Knochensubstanz sowie die Heilung im Wundbereich födert.

Bei den mit aufnehmerspule und Elektrodenanschlüssen versehenen Osteosyntheseimplantaten der oben genannten Art, die im folgenden als "Elektro-Osteosyntheseimplantate" bezeichnet werden sollen, handelt es sich um Spezialkonstruktionen, bei denen speziell bearbeitete Knochenplatten bzw. Knochenmarknägel benötigt werden, mit denen die Aufnehmerspule und Elektroden bzw. Elektrodenanschlüsse durch Klebung, Klemmverbindungen und/oder Verschweissungen fest verbunden sind. Um für den Bedarfsfall gerüstet zu sein, benötigten die chirurgischen Abteilungen der orthopädischen Kliniken dementsprechend ein Lager mit Elektro-Osteosyntheseimplantaten der verschiedensten Typen, bei Knochenmarknägeln z.B. des gesamten Längen- und Durchmesserspektrums. Dies bedeutet eine finanziell kaum zumutbare Mehrbelastung des ohnehin meist kritischen Haushaltes einer Klinik. Elektro-Osteosyntheseimplantate wurden daher häufig erst bei Nachoperationen eingesetzt und dann für den jeweiligen Fall beim Hersteller einzeln bestellt.

Aus der oben bereits benannten CH—A—551 201 ist zwar bereits eine Cerclage bekannt geworden, welche einen gürtelartigen Teil, an dem Elektroden angebracht sind, und einen schließenartigen Teil, an dem eine Aufnehmerspule angebracht ist, aufweist. Eine solche Cerclage kann nach Implantierung eines Knochenmarnagels um den Knochen gelegt und die Aufnehmerspule kann mit einem Wicklungsanschluß mit dem Knochenmarknagel verbunden werden. Weiterhin ist aus der DE—A—23 11 817 eine lose implantierbare Aufnehmerspule bekannt, die in einen gewebeverträglichen Kunststoff eingebettet ist und flexible Elektrodenanschlüsse aufweist, die über

druckknopfartige Anschlußvorrichtungen mit entsprechend ausgebildeten Knochenschrauben, die dann als Elektroden dienen, verbunden werden können. Aus der US—A—3 918 440 ist es bekannt, eine solche lose Aufnehmerspule in Kombination mit Knochenschrauben zu verwenden, deren Kopf isoliert ist, so daß sie zur Befestigung einer Knochenplatte verwendet werden können und dann Elektroden bilden, die bezüglich der Knochenplatte isoliert sind.

Weder die Cerclage noch der lose Übertrager mit den über druckknopfartige Verbindungsmittel anschließbaren Knochenschrauben stellen jedoch einen vollwertigen Ersatz für ein Elektro-Osteosyntheseimplantat der oben angegebenen Art dar. Die Verwendung einer Cerclage bedeutet außerdem eine zusätzliche Komplizierung. Die lose Aufnehmerspule ist schwierig zu fixieren.

Der vorliegenden Erfindung liegt dementsprechend die Aufgabe zugrunde, eine Zusatzvorrichtung anzugeben, durch die ein gewöhnliches Osteosyntheseimplantat, wie eine Knochenplatte oder ein Knochenmarknagel jederzeit, also auch während einer Operation, in ein vollwertiges Elektro-Osteosyntheseimplantat umgewandelt werden kann.

Die Erfindung, wie sie in den Ansprüchen gekennzeichnet ist, löst diese Aufgabe durch Schaffung einer Zusatzvorrichtung, mit der ein konventionelles Osteosyntheseimplantat, wie eine Knochenplatte oder ein Marknagel (Küntscher-Nagel) aus Metall, Keramik und dergl., jederzeit, also auch im Verlauf einer Operation in ein vollwertiges Elektro-Osteosyntheseimplantat umgewandelt werden kann. Die Vorratshaltung der Klinik an Osteosyntheseimplantaten wird dadurch erheblich erleichtert und verbilligt.

Die vorliegende Zusatzvorrichtung ermöglicht es dem Chirurgen, eine Operation zur Versorgung eines verletzten oder anderweitig beschädigten Knochens unter Verwendung von vorrätigen, üblichen Osteosyntheseimplantaten in gewohnter Weise durchzuführen. Er kann dann vor dem Verschließen des Weichgebietes entscheiden, ob die Verwendung eines Elektro-Osteosyntheseimplantats indiziert ist. Wenn er die Verwendung eines Elektro-Osteosyntheseimplantats für angezeigt hält, kann er eine am Knochen angebrachte Knochenplatte oder einen in einen Knochen eingesetzten Knochenmarknagel mit der vorliegenden Zusatzvorrichtung versehen.

Im Falle eines Knochenmarnagels kann die Nagelung des gebrochenen Knochen in üblicher Weise mit einem Führungsspieß ausgeführt werden, da die Zusatzvorrichtung gemäß der Erfindung eine vom Marknagel unabhängige Einheit darstellt, die nach der Implantation des rohrförmigen, längsgeschlitzten Nagels längs des Schlitzes in das Innere des Nagels eingeschoben werden kann und dann ohne weitere Maßnahmen betriebsbereit ist. Die Zusatzvorrichtung läßt sich dabei so ausbilden, daß sie für Knochenmarknägel unterschiedlicher Durchmesser (siehe z.B. DIN-Blatt 58801) verwenbar ist.

Ein gewöhnliches Osteosyntheseimplantat (Knochenstützplatte) Knochenmarknagel ohne Aufnehmerspule und Elektroden) und die Zusatzvorrichtung gemäß der Erfindung sind zusammen außerdem erheblich einfacher und kostengünstiger herzustellen als die in den oben erwähnten Patentschriften beschriebenen Elektro-Osteosyntheseimplantate.

Im folgenden werden Ausführungsbeispiele der Erfindung unter Bezugnahme auf die Zeichnung näher erläutert:

Es zeigen:

Fig. 1 eine Draufsicht auf einen Knochenmarknagel nach Küntscher gemäß DIN 58801, Form B;

Fig. 2 einen vereinfachten Längsschnitt einer Zusatzvorrichtung, mit der der Knochenmarknagel gemäß Fig. 1 in ein Elektro-Implantat verwandelt werden kann;

Fig. 3 einen Querschnitt in einer Ebene III—III der Fig. 2;

Fig. 4 eine Draufsicht auf einen Teil eines Knochenmarknagels der in Fig. 1 dargestellten Art mit eingeschobener Zusatzvorrichtung ähnlich der gemäß Fig. 2 and 3;

Fig. 5 ein Querschnittsansicht in einer Ebene V—V der Fig. 4;

Fig. 6 eine Seitenansicht einer Abwandlung der Zusatzvorrichtung gemäß Fig. 4 und 5;

Fig. 7 eine schematische Darstellung einer elektrischen Schaltung der Aufnehmerspule und Elektroden der Zusatzvorrichtung gemäß Fig. 6;

Fig. 8 eine schematische perspektivische Darstellung einer für eine Osteosyntheseplatte geeigneten Ausführungsform der vorliegenden Zusatzvorrichtung;

Fig. 9 eine Draufsicht auf eine andere Ausführungsform der Erfindung fuür eine Osteosyntheseplatte;

Fig. 10 und 11 Draufsichten einer elastischen Elektrodenanschlußhalterung im ungedehnten bzw. gedehnten Zustand;

Fig. 12 eine Seitenansicht einer Zusatzvorrichtung für einen Knochenmark- oder Küntscher-Nagel gemäß einer weiteren Ausführungsform der Erfindung;

Fig. 13 eine Draufsicht auf ein Ende der Zusatzvorrichtung gemäß Fig. 12, gesehen in Richtung von Pfeilen XIII—XIII;

Fig. 14 eine perspektivische Ansicht eines Teiles eines Marknagels, in den eine Zusatzvorrichtung gemäß Fig. 12 eingesetzt ist;

Fig. 15 eine vergrößerte Querschnittsansicht der Zusatzvorrichtung in einer Ebene XIV—XIV der Fig. 12 und des Marknagels;

Fig. 16 eine teilweise längsgeschnittene Seitenansicht eines Endes einer Zusatzvorrichtung gemäß einer weiteren Ausführungsform der Erfindung;

Fig. 17 eine Schnittansicht der Zusatzvorrichtung gemäß Fig. 16 in einer Ebene XVII—XVII und eines Marknagels, in den die Zusatzvorrichtung eingesetzt ist;

Fig. 18 eine Draufsicht auf eine Abwandlung der Zusatzvorrichtung gemäß Fig. 12 bis 15;

Fig. 19 eine Seitenansicht einer weiteren Abwandlung der Ausführungsform gemäß Fig. 2 bis 5;

Fig. 20 eine perspectivische Ansicht des vorderen Endes der Zusatzvorrichtung gemäß Fig. 19;

Fig. 21 ein Querschnitt in einer Ebene XXI—XXI der Fig. 19;

Fig. 22 einen vergrößerten Längsschnitt in einer Ebene XXIII—XXIII der Fig. 20;

Fig. 23 einen vergrößerten Längsschnitt des vorderen Ends der Zusatzvorrichtung gemäß Fig. 19 in einer Ebene XXIII—XXIII in Fig. 19 und

Fig. 24 eine Abwandlung eines Teiles der Zusatzvorrichtung gemäß Fig. 19.

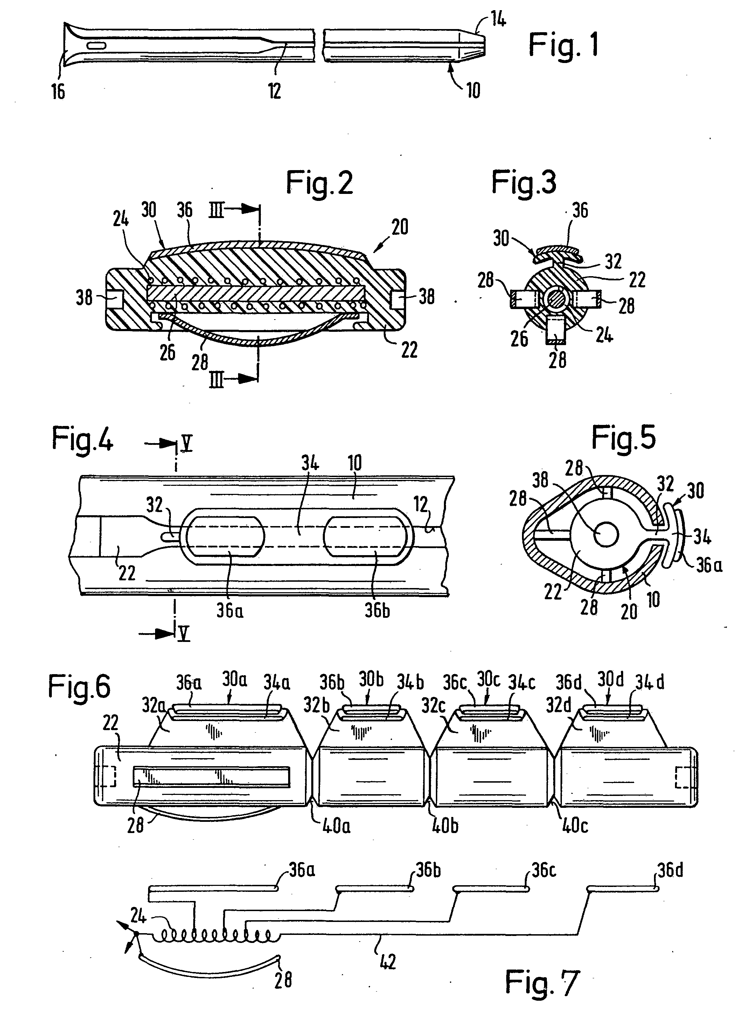

Fig. 1 zeigt einen Knochenmarknagel 10, der der DIN-Norm 58801, Form B entspricht und als bekannt vorausgesetzt werden kann. Er hat einen etwa kleeblattförmigen Querschnitt (siehe Fig. 5), einen Längsschlitz 12, eine etwas zulaufende, verjüngte Spitze 14 und ein etwas erweitertes Ende 16.

Der konventionelle Knochenmarknagel 10 kann durch eine Zusatzvorrichtung 20, deren Konstruktion insbesondere aus den Fig. 2 und 3 ersichtlich ist, zu einem Elektroimplantat ergänzt werden, mit dem der verletzte Knochenbereich elektrodynamisch stimuliert und zu einer beschleunigten Knochenneubildung und Heilung angeregt werden kann. Die Zusatzvorrichtung 20 hat einen Körper 22 aus gewebeverträglichem Kunststoff, wie Polyäthylen oder einem Kohlenstoff-Fluor-Polymerisat, in den eine solenoidförmige Aufnehmerspule 24 eingebettet ist. Die Aufnehmerspule 24 ist in den Figuren 2 und 3 nur schematisch als einlagige Spule dargestellt, sie kann selbstverständlich mehrere, eng gewickelte Wicklungslagen enthalten. Die Schaltung und elektrischen Anschlüsse der Spule sind in den Figuren 2 und 3 der Einfachheit halber nicht dargestellt, auf sie wird noch später eingegangen werden.

Die Aufnehmerspule 24 enthält vorzugsweise einen Magnetkern 26, der aus einem magnetisch weichen Material, wie einem Ferrit oder einer Eisen-Nickel-Legierung bestehen kann. Man kann jedoch auch ein Permanentmagnetmaterial verwenden, das dann vorzugsweise in einen teilweise gesättigten Zustand gebracht wird und die Kurvenform der den Elektroden zugeführten Spannungen und Ströme zu modification gestattet.

Bei dem in Fig. 2 und 3 dargestellten Ausführungsbeispiel der Zusatzvorrichtung sind im Körper 22 drei blattfederartige Elektroden 28 gelagert, die bogenförmig nach außen gewölbt sind. Die Elektroden 28 sind elektrisch miteinander und einem Windungsanschluß der Wicklung der Aufnehmerspule 24 verbunden und dienen zum Anschluß der Zusatzvorrichtung an den aus Metall bestehenden Körper des Knochenmarknagels 10, wie es in Fig. 5 dargestellt ist. Vorzugsweise sind die Abmessungen des Körpers 22 und der Federweg der Elektroden 28 derart bemessen, daß ein und dieselbe Zusatzvorrichtung 20 für Knochenmarknägel des ganzen praktisch vorkommenden Durchmesserbereiches oder zumindest einen großen Teil des Durchmesserbereiches verwendet werden kann.

Der Körper 22 weist ferner einen im Querschnitt etwa T-förmigen Ansatz 30 auf, der so bemessen ist, daß der Stegteil 32 (Fig. 5) in den Schlitz 12 paßt. Der obere Querteil 534 des Ansatzes kann, wie insbesondere aus Fig. 5 ersichtlich ist, etwas gebogen sein und sich der Form der Außenfläche des Knochenmarknagels 10 anpassen. Der Querteil 34 kann in Längsrichtung des Nagels gesehen entweder flach (Fig. 4 und 5) oder gebogen (Fig. 2 und 3) sein. Er trägt ein Elektrode 36, die mit einer anderen Stelle der Aufnehmerspule 24 als die Elektroden 28 verbunden ist und als Gegen- oder Gewebeelektrode dient.

Wie Fig. 4 zeigt, können auf dem Ansatz 30 auch mehrere, elektrisch getrennte und mit verschiedenen Windungsanschlüssen der Spule 24 verbundene Gewebeelektroden 36a, 36b usw. vorgesehen sein.

Der Körper 22 kann an seinen Enden mit durch Vertiefungen 38 angedeuteten Mitteln zum Anbringen eines Werkzeuges zum Manipulieren, z.B. Herausziehen der Zusatzvorrichtung aus dem Knochenmarknagel, versehen sein.

Vorzugsweise wird dem Chirurgen mit der Zusatzvorrichtung 20 noch eine Füll-Leiste aus gewebeverträglichem Kunststoff mitgeliefert, die z.B. ein Doppel-T-Profil haben kann und so bemessen ist, daß sie nicht durch die verjüngte Spitze 14 des Nagels austreten kann. Diese Füll-Leiste kann vom Chirurgen auf die gewünschte Länge zugeschnitten und zusammen mit der Zusatzvorrichtung 20 in den Nagel 10 eingeführt werden, um einen gewünschten Abstand der Zusatzvorrichtung 20 von der Spitze 14 des Nagels 10 sicherzustellen und/oder bei Verwendung mehrerer Zusatzvorrichtungen 20 einen gewünschten Abstand zwischen diesen zu gewährleisten.

Der Körper 22 mit dem Ansatz 30 und den Elektroden 36 bzw. 36a, 36b sind so bemessen, daß die in den Knochenmarknagel 10 eingeführte Zusatzvorrichtung (Fig. 5) in den Bohrkreisdurchmesser des chirurgischen Bohrwerkzeuges paßt, mit dem das Loch für den Knochenmarknagel ausgebohrt wird.

Die gesamte Länge der Zusatzvorrichtung 20 kann etwa 30 bis 50mm betragen und ist, gegebenenfalls in Verbindung mit einer gewissen Elastizität des Materiales des Körpers 22 so bemessen, daß die Zusatzvorrichtung mühelos über einen Knick vom oberen Ende eines gebogenen Knochmarknagels in den dort erweiterten Längsschlitz 12 eingeführt werden kann. Der Außbendurchmesser der Zusatzvorrichtung ist größer als die untere Öffnung des zur Spitze 14 konisch zulaufenden Knochenmarknagels, so daß ein Durchgleiten der Zusatzvorrichtung beim Einschieben oder beim Ziehen des Nagels sicher vermieden wird.

Der Knochenmarknagel 10 ist über die Elektroden 28 galvanisch mit der Aufnehmerspule 24 verbunden und bildet eine der am Knochen anleigenden Elektroden. Die andere Elektrode oder anderen Elektroden 36a, 36b können einen galvanischen, halbleitenden oder kapazitiven Kontakt mit dem an sie anliegenden Gewebe des Patienten machen und in den letzten beiden Fällen mit entsprechenden Überzügen versehen sein. Bei schlecht durchblutetem Gewebe mit wenigen Gefäßen ein galvanischer Kontakt zweckmäßig. In besser durchblutetem Gewebe kann ein halbleitender kontakt zweckmäßig sein, der z.B. mittels einer dünnen Beschichtung, z.B. aus $Al_2O_3$, Biokohle, Calciumphosphat u. dgl. realisiert werden kann. Bei starker Vaskularisation des kontaktierten Gewebes kann ein kapazitiver Kontakt zweckmäßig sein, da hierdurch ein Ansteigen des Stromes über die physiologisch verträgliche Grenze von ca. 100 Mikroampere vermieden wird, was bei galvanischem Kontakt infolge des hohen Leitwertes von stark vaskularisiertem Gewebe unter Umständen eintreten kann.

Fig. 6 zeigt eine Seitenansicht einer gegenüber Fig. 4 etwas abgewandelten Ausführungsform der vorliegenden Zusatzvorrichtung, die hier vier Gewebeelektroden 36a bis 36d enthält. Der die Gewebeelektroden tragende Ansatz ist in vier Teile 30a, 30b, 30c, und 30d unterteilt. Der Körper 22 weist zwischen den einzelnen Ansätzen Einschnürungen 40a, 40b bzw. 40c auf. Wie Fig. 7 zeigt, ist das eine Ende der Aufnehmerspule 24 mit den Elektroden 28 verbunden, die zum Anschluß der Aufnehmerspule an den Knochenmarknagel bestimmt sind und so angeordnet und ausgebildet sein können, wie es anhand der Figuren 2 und 3 erläutert worden ist. Die Gewebeelektroden 36a bis 36d sind mit Windungsanschlüssen der Aufnehmerspule 24 verbunden, welche zunehmend größere Abstände von dem mit den Elektroden 28 verbundenen Ende der Aufnehmerspule 24 haben.

Die Zusatzvorrichtung gemäß Fig. 6 kann vom Chirurgen durch Abschneiden oder Abzwicken des Körpers 22 bei einer der Einschnürungen 40a bis 40c der Ausdehnung des Frakturbereiches angepaßt werden. Die Gewebeelektroden 36b bis 36d sind mit der Aufnehmerspule 24 über Verbindungsleitungen 42, z.B. in Form dünner Folein, verbunden, die sich leicht abtrennen lassen. (Fig 7.)

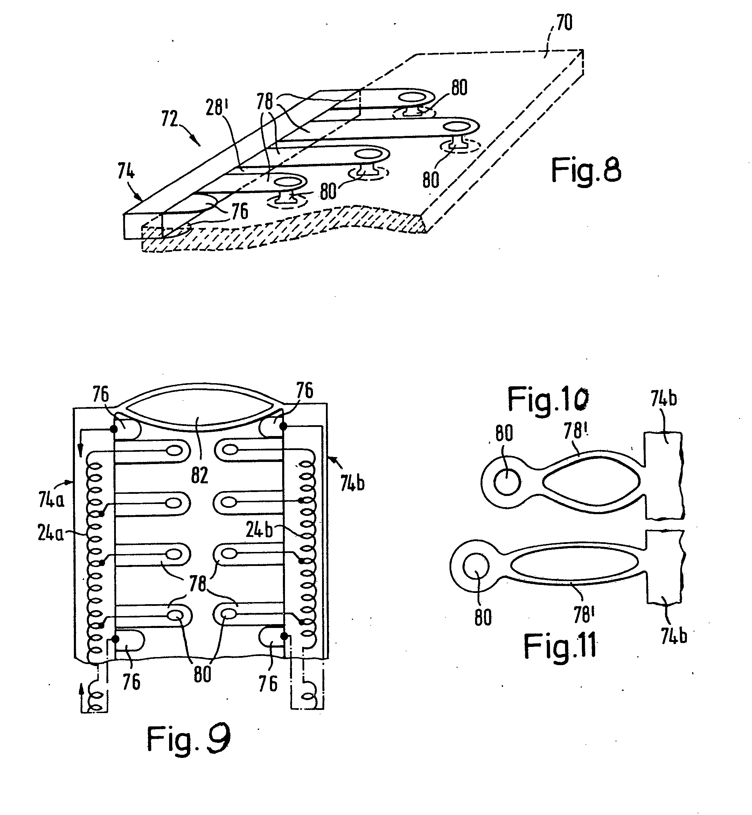

Fig. 8 zeigt eine Ausführungsform der Zusatzvorrichtung gemäß der Erfindung, die zum Anbringen an eine nur schematish, gestrichelt dargestellte Knochenplatte 70 bestimmt ist. Die Zusatzvorrichtung 72 gemäß Fig. 8 enthält einen Körper 74 aus einem gewebeverträglichen Kunststoff, in dem ähnlich wie im Körper 22 eine nicht dargestellte Aufnehmerspule angeordnet ist. Der Körper hat vorzugsweise einen annähernd rechteckigen Querschnitt mit einer Dicke, die die der Knochenplatte 70 nicht nennenswert übersteigt. Von der einen Seite des Körpers springen ein oder mehrere Paare von messerkontaktartigen, vorzugsweise federnden Halterungteilen 76 vor, die sowohl zur elektrischen Kontaktierung als auch zur mechanischen Halterung des Körpers 74 an der Knochenplatte 70 dienen. Die Halterungstiele 76 stellen ähnlich wie die Elektroden 28 den elektrischen Kontakt zwischen der Aufnehmerspule und der Platte 70 her. Zustätzlich kann noch eine federnde Elektrode 28' vorgesehen sein, die ähnlich wie die Elektroden 28 der Ausführungsform gemäß Fig. 2 und 3 ausgebildet ist und mit einer Seitenfläche der Knochenplatte 70 Kontakt macht, wenn die Zusatzvorrichtung 72 an der Knochenplatte angebracht ist. Vom Körper 74 springen ferner bandförmige Halterungsteile 78 vor, die aus Kunststoff bestehen und an den Körper 74 angeformt sein können. Die Halterungsteile 78 tragen : Anschlußvorrichtungen 80, die über in die Halterungsteile 78 eingebettete Leitungen mit der Aufnehmerspule verbunden sind, ähnlich wie die Elektroden 36a bis 36d in Fig. 6. Die Anschlußvorrichtungen 80 dienen zum Anschluß der Aufnehmerspule an nicht dargestellte, isolierte Knochenschrauben, die gleichzeitig als Elektroden dienen und so ausgebildet sein können, wie es in der oben erwähnten US-Patentschrift dargestellt ist. Nicht benötigte Halterungsteile 78 werden abgeschnitten. Die Halterungsteile können, wie dargestellt, unterschiedliche Längen haben und so ausgebildet sein, daß die Anordnung der Anschlußvorrichtungen 80 der Verteilung der Löcher in einer Standard-Knochenplatte entspricht.

Fig. 9 zeigt eine Weiterbildung der Ausführungsform gemäß Fig. 8, die zwei Körper 74a, 74b, aufweist, die jeweils eine Aufnehmerspule 24a bzw. 24b enthalten. Die nicht dargestellten, in Fig. 9 unteren Enden beider Aufnehmerspulen sind mit den zum Anschluß an die Platte dienenden, als Elektroden ausgebildeten Halterungsteilen 76 (und gegebenenfalls den Elektroden 28) verbunden und die Aufnehmerspulen 24a können entweder gleichsinnig oder gegensinnig gewickelt sein, so daß die Anschluß vorrichtungen 80 gegenüberliegender Halterungsteile 78 gleiche oder unterschiedliche Potentiale führen. Die beiden Körper 74a, 74b sind zumindest and den Enden über eine Art Feder 82 aus Kunststoff verbunden, so daß die Zusatzvorrichtung gemäß Fig. 9 für Platten verschiedener Breite verwendet werden kann.

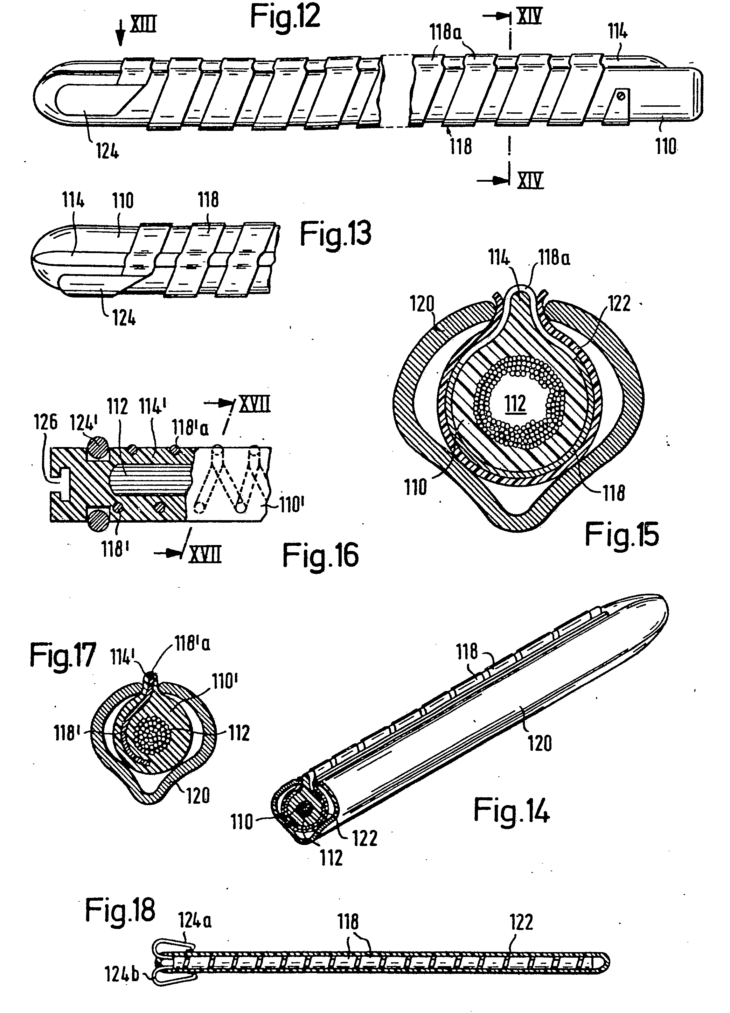

Die die Anschlußvorrichtungen 80 tragenden Halterungsteile können vorteilhafterweise so ausgebildet sein, wie es in Fig. 10 und 11 durch die Halterungsteile 78' dargestellt ist. Diese Halterungsteile 78' bestehen aus zwei Streifen eines elastischen Kunststoffs, die im Ruhezustand gebogen sind, wie es in Fig. 10 dargestellt ist. Die Streifen wirken als Feder, so daß die Lage der Anschlußvorrichtungen 80 unterschiedlichen Abständen zwischen dem Rand der Platte und den Knochenschrauben, mit denen die Anschlußvorrichtungen 80 Kontakt machen, angepaßt werden kann.

Bei Verwendung der Zusatzvorrichtungen gemäß Fig. 8 und 9 wird zuerst die Knochenplatte 70 am Knochen befestigt, wobei uberall dort isolierte Knochenschrauben, deren Kopf als zu den Anschlußvorrichtungen 80 komplementäres Anschlußelement ausgebildet ist, verwendet werden, wo Elektroden nötig sind. Dann wird die Zusatzvorrichtung auf die Längsränder der Platte gesteckt und die isolierten Knochenschrauben werden mit Anschlußvorrichtungen 80 verbunden. Die nicht benötigten Anschlußvorrichtungen werden mit den sie tragenden Halterungsteilen abgeschnitten.

Die Zusatzvorrichtungen gemäß der Erfindung ermöglichen es dem Chirurgen also, erst im Laufe der Operation zu entscheiden, ob eine fragliche Heiltendenz des Patienten und/oder die Schwere der Verletzung eine elektrodynamische Aktivierung der Osteosynthese als wünschenswert erscheinen läßt.

Wenn die vorliegenden Zusatzvorrichtungen für ein Osteosyntheseimplantat aus einem nichtleitenden Material, wie Keramik, verwendet werden sollen, wird anstelle der den Implantatkörper kontaktierenden Elektrode oder Elektroden eine (oder mehrere) großflächige Elektrode vorgesehen, die mit dem Gewebe, vorzugsweise dem den Knochen umgebenden Weichgewebe in Berührung steht. Bei der Ausführungsform gemäß Fig. 4 oder 5 kann dann z.B. eine der Elektroden 36 als diese Gewebeeelektrode verwendet werden.

Als Körper kann auch eine Art Gehäuse aus einem gewebeverträglichen Metall, wie einer Kobalt-Chrom-Legierung, Chrom-Nickel-Manganstahl, Titan und dergleichen verwendet werden. Der Körper kann dans als Elektrode zum Anschluß an ein metallisches Osteosyntheseimplantat dienen oder eine großflächige Gewebeeelektrode bilden. Die anderen Elektroden werden dann in geeigneter Weise gegen den Körper isoliert.

Bei den in den Figuren 12 bis 18 dargestellten Zusatzvorrichtungen, die fur Knochenmarknägel der in Fig 1 dargestellten Art bestimmt sind, ist ein Teil mindestens einer Windung der Wicklung der Aufnehmerspule aus einer elektrisch isolierenden Umhüllung aus gewebeverträglichem Material herausgeführt und so an der Oberfläche der Umhüllung angeordnet ist, daß der betreffende Teil der Windung der Elektrode für das Gewebe wirkt, welches bei implantiertem und mit der Zusatzvorrichtung versehenem Osteosyntheseimplantat letzteres umgibt.

Bei diesen Ausführungsformen der Erfindung besteht also mindestens eine Gewebeelektrode aus einem Teil einer Windung der Wicklung der Aufnehmerspule. Die Aufnehmerspule kann zweckmäßigerweise eine einlagige Wicklung aus einem bandförmigen Leiter enthalten und im wesentlichen jede Windung kann einen als Gewebeelektrode wirkenden Teil enthalten. Vorzugsweise ist die Wicklung an einem Punkt, z.b. in der Mitte, vorzugsweise jedoch an einem Ende, mit dem Osteosyntheseimplantat verbunden, das aus Metall besteht oder eine oberflächliche metallschicht aufweist so daß das Implantat ebenfalls als Elektrode wirkt.

Die in den Fig. 12 bis 15 dargestellte Zusatzvorrichtung besteht aus einem langgestreckten stabartigen Körper 110 aus einem beigsamen, gewebeverträglichen isolierenden Material, wie PTFE. Der Körper 110 enthält vorzugsweise einen Magnetkern 112, z.B. aus einem hochpermeablen, magnetisch weichen Material, wie Mumetall oder Permalloy. Damit der Körper 110 biegsam ist und auch in abgebogene Marknägel eingeführt werden kann, besteht der Kern vorzugsweise aus einem Bündel von Drähten aus dem betreffenden ferromagnetischen Werkstoff, wie in Fig. 15 dargestellt ist.

Der Körper 110 hat einen in seiner Längsrichtung verlaufenden leistenartigen, abgerundeten Vorsprung 114, der dem Querschnitt des Körpers 110 eine näherungsweise birnenförmige Gestalt verleiht (Fig. 15). Auf dem Körper 110 ist eine näherungsweise wendelförmige Wicklung 118 aus einem bandförmigen Leiter angeordnet, die sich der Oberfläche des Körpers anpaßt. Der bandförmige Leiter besteht zumindest an den Teilen seiner Oberfläche, die mit Gewebe- oder Körperflüssigkeit in Berührung kommen können, aus einem biologisch verträglichen Metall. Vorzugsweise besteht der bandförmige Leiter aus Osteosynthesemetall, also z.B. einer Kobalt-Chrom-Molybdän-Legierung, die unter dem Handelsnamen "Vitallium" bekannt ist.

Die Länge des Körpers 110 Kann z.B. 250 mm betragen. Der bandförmige Leiter kann beispeilsweise 3 bis 6 mm breit und beispielsweise 0,1 mm dick sein. Die Wicklung kann beispielsweise zehn bis fünfzig Windungen enthalten.

Bei einer praktischen Ausführungsform bestand die Wicklung 118 aus einem 5 mm breiten und 0,1 mm dicken bandförmigen Leiter aus Osteosynthesemetall gemäß DIN 4401 und enthielt etwa 32 Windungen. Der Abstand zwischen den Rändern benachbarter Windungen betrug etwa 0,8 mm. Die Enden der Wicklung sind am Körper 110 festgelegt, z.B. durch Einbetten, durch eine Schraube oder dergl.

Die Querschnittsabmessungen des mit der Wicklung 118 versehenen Körpers 110 sind so gewählt, daß der Körper mit der Wicklung und eine aus einer Folie bestehenden Umhüllung 122 (Fig. 15) leicht in die hintere Öffnung eines vorgegebenen Marknagels 120 axial eingeschoben werden können und in diesem gut passend sitzen.

Die isolierende Umhüllung 122 besteht aus einer Folie aus gewebeverträglichem Kunststoff und hat die Aufgabe, die Wicklung 118 zumindest über den größten Teil der Länge der Wicklung gegen den metallischen Marknagel 120 zu isolieren. Die Umhullung 122 kann entfallen, wenn der Marknagel aus einem isolierenden Werkstoff besteht oder die Isolation auf andere Weise gewährleistet ist. Eine etwa 0,1 mm dicke PTFE-Folie hat sich also Umhüllung 122 bewährt. Die Umhüllung kann an dem zuerst in den Marknagel eingeführten Ende mit dem Körper 110 verbunden werden oder ein kappenartig geschlossenes Ende aufweisen, um zu verhindern, daß die Umhüllung 122 beim Einschieben der Zusatzvorrichtung in den Marknagel sich bezüglich des Körpers 110 verschiebt.

Die Wicklung 118 kann vollständig gegen den Marknagel 120 isoliert sein, vorzugsweise wird sie jedoch an einem Punkt, z.B. in der Mitte oder vorzugsweise an dem zuletzt eingeführten Ende elektrisch mit dem Marknagel 120 verbunden, wenn dieser aus Metall besteht. Die Wicklung 118 kann hierzu ein als anschlußkontakt 124 dienendes, laschenartiges Ende aufweisen, das von der Umhüllung 122 nicht abgedeckt wird und nach Einschieben der Zusatzvorrichtung in den Marknagel mit dessen Innenwand Kontakt macht.

Nach der Implantation wird in der Wicklung 118 eine niederfrequente Wechselspannung mit einer Frequenz von vorzugsweise unter 30 Hz induziert, wie es bei Elektro-Implantaten der eingangs genannten Art im Prinzip bekannt ist. Mit magnetischen Feldstärken in der Gröbenordnung von $10^{-2}$ Tesla lassen sich in der Wicklung 118 Wechselspannungen in der Größenordnung von einigen 100 Millivolt induzieren. Die niederfrequente Wechselspannung wird zwischen den freiliegenden Teilen 118a der Windungen der Wicklung 118, die als Gewebeelektroden wirken, wirksam und regt das Wachstum des angrenzenden Knockengewebes in bekannter Weise an. Der Widerstand der Wicklung 118 ist vorzugsweise gering, insbesondere unter 30 oder 10 Ohm, was durch den großen Querschnitt des bandförmigen Leiters der Wicklung 118 gewährleistet ist, do daß der elektrische Nebenschluß durch das Gewebe, das an den Windungsteilen 118a anliegt, praktisch vernachläßigt werden kann.

Wenn das zuletzt in den Marknagel eingeführte Ende der Wicklung 118 durch den Anschlußkontakt 124 mit dem aus Metall bestehenden Marknagel verbunden wird, wirkt der Marknagel als Gegenelektrode und die Potentialdifferenz zwischen dem Marknagel10 (Fig. 1) und den Windungsteilen 118a nimmt vom hinteren Ende der Wicklung (in Fig. 12 links) zum vorderen Ende hin zu. Dies hat den Vorteil, daß in der Mitte des Nagels und an seinem distalen Ende die größe Spannungsdifferenz wirksam wird, wo im allgemeinen auch die Verletzungen am häufigsten sind.

Fig. 18 zeigt etwas vereinfacht eine Abwandlung der Ausführungsform gemäß Fig. 1 bis 4; der einzige Unterschied besteht darin, daß am proximalen Ende der Wicklung 118 zwei bügelartige, federnde Anschlußkontakte 124a und 124b angebracht sind, die mit der Innenseite des zugehörigen Marknagels (Fig. 1) elektrisch Kontakt machen, wenn die Zusatzvorrichtung in den Marknagel eingeschoben ist.

Bei allen Ausführungsformen können die als Gewebeelektroden dienenden Teile 118a der Wicklung 118 auch mit einer dünnen isolierenden oder halbleitenden Schicht überzogen werden, um die am Gewebe wirksam werdende Spannung zu steuern. Man kann durch diese Maßnahme auch die Spannung vergleichmäßigen, z.B. indem man bei einer am proximalen Ende des Nagels an diesen angeschlossenen Wicklung 118 auf die als Gewebeelektroden wirkenden Teile 118a der Wicklung im mittleren Wicklungsbereich eine halbleitende und im distalen Wicklungsbereich, wo die Spannung am höchsten ist, eine dünne isolierende Schicht aufbringt.

Weiterhin können zwischen die Wicklung 118 und den als Elektroden verwendeten Marknagel ein ggfs. mit einem Widerstand überbrückter Gleichrichter geschaltet werden, um die Null-Linie der induzierten Wechselspannung zu verschieben, d.h. daß dann eine unsymmetrische Wechselspannung oder eine mit einer Wechselspannung überlagerte Gleichspannung zur Einwirkung gebracht wird.

Die in den Fig. 16 und 17 teilweise dargestellte Ausführungsform der Erfindung unterscheidet sich von der gemäß Fig. 12 bis 15 in erster Linie darin, daß die Wicklung 118' in den aus gewebeverträglichem Kunststoff wie PTFE bestehenden Körper 110' so eingebettet ist, daß lediglich die als Gewebeelektroden dienenden Kuppen 118' a der Windungen an der Oberseite des leistenartigen Vorsprungs 114' des Körpers freiliegen. Der Körper 110' dient also gleichzeitig als isolierende Umhüllung für den größten Teil der Wicklung, so daß die bei der Ausführungsform gemäß Fig. 12 bis 15 als Umhüllung 122 dienende Folie entfallen kann. Das in Fig. 16 linke Ende der Wicklung 118' ist in nicht dargesteller Weise elektrisch mit einem Anschlußkontakt 124' verbunden, der die Form eines Federringes hat. Das hintere Ende des Körpers 110' ist außerdem mit einer Ausnehmung 126 versehen, die beispielsweise eine schlitzförmige Öffnung hat, in die ein entsprechend geformtes Werkstück zum Einschieben bzw. Herausziehen der Zusatzvorrichtung eingesetzt werden kann. Der die Wicklung 118' bildende Leiter hat bei der Ausführungsform gemäß Fig. 16 und 17 einen kreisförmigen Querschnitt und die in den Vorsprung 114' hineinreichenden, die Gewebeelektroden bildenden Windungsteile sind haarnadelförmig gebogen, wie Fig. 17 zeigt. Der Querschnitt des leiters der Wicklung 118' soll wieder so groß sein, daß der Nebenschluß durch das Gewebe kein nennenswertes Zusammenbrechen der induzierten Spannung verursachen kann.

Bei geeigneter Form und Elastizität des Körpers 110 läßt sich die Zusatzvorrichtung für Marknägel mehrerer verschiedener Größen verwenden. Die Zusatzvorrichtung kann außerdem als "Meterware" hergestellt werden, von der der Chirurg dann Stücke der gewünschten Länge abschneiden kann. Dabei kann man dann entweder auf einen Anschluß der Zusatzvorrichtung an den Nagel verzichten oder im Falle der Ausführungsform gemäß Fig. 12 bis 15 einfach die Umhüllung 122 etwas kürzer bemessen als die Wicklung 118, so daß ein Ende der Wicklung Kontakt mit dem Nagel machen kann, oder man löst ein Ende der Wicklung 118' der Zusatzvorrichtung gemäß Fig. 16 und 17 aus dem Körper 110' heraus und legt es so um die Außenseite des Körpers, daß es nach Einsetzen der Zusatzvorrichtung in den Marknagel mit dessen Innenwand Kontakt macht.

Auch bei den unter Bezugnahme auf die Fig. 9 bis 11 beschriebenen Zusatzvorrichtungen für Osteosyntheseplatten kann mindestens eine Gewebeelektrode durch einen Teil einer Windung der Wicklung einer Aufnehmerspule gebildet werden.

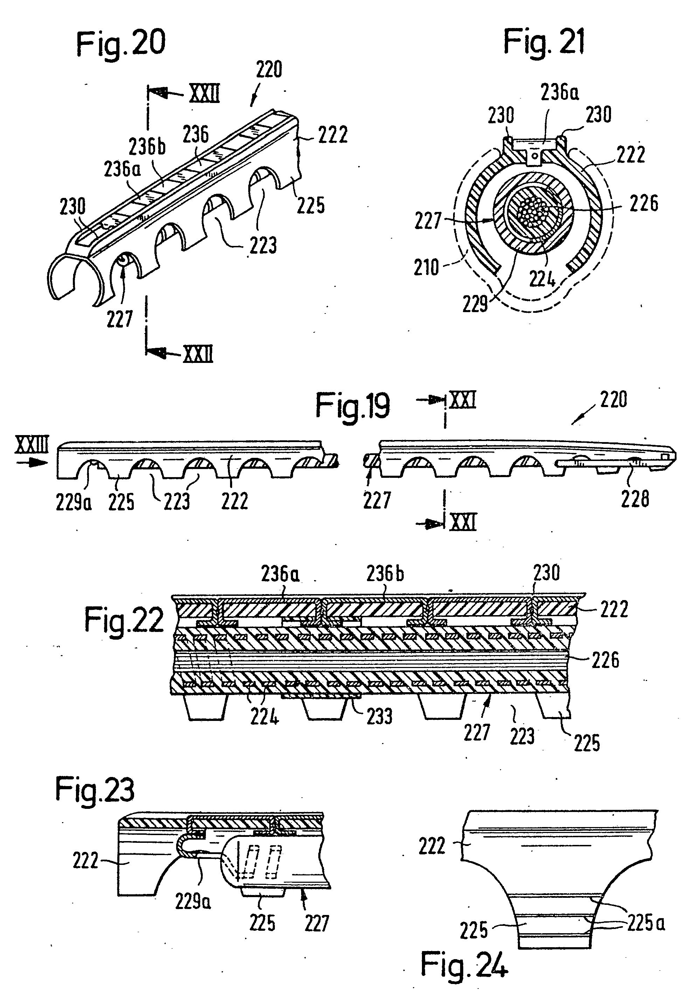

In den Fig. 19 bis 23 ist eine Zusatzvorrichtung 220 dargestellt, die zum Einschieben in einen Knochenmarknagel bestimmt ist. Der Knochenmarknagel kann einen durchgehenden Längsschlitz haben, wie der in Fig. 1 dargestellte Knochenmarknagel oder er kann am hinteren Ende auch die Form eines geschlossenen Rohres haben (AO-Modell). Die Zusatzvorrichtung 220 hat einen langgestreckten Körper 222 aus einem biologisch verträglichen isolierenden Kunststoff oder dergleichen, z.B. Polyäthylen oder PTFE. Der Körper 222 kann beispielsweise aus einem Polyäthylen-Spritzgußteil bestehen. Er hat einen ungefähr hufeisenförmigen Querschnitt (Fig. 21) und ist oben in der Mitte mit zwei Längsrippen 230 versehen, die eine langgestreckte kanalartige Vertiefung bilden, in der eine Elektrodenanordnung 236 angeordnet ist. Die Seitenteile des Körpers 222 sind mit halbkreisförmigen Ausschnitten 233 versehen, die die Biegsamkeit des Körpers erhöhen und als Federn wirkende Laschen 225 begrenzen, die bei Gebrauch gegen die Innenwand des Marknagels 210 (Fig. 21) drücken und die Zusatzvorrichtung im Marknagel fixieren. Die Laschen 225 können mit Längsnuten 225a versehen sein, wie es in Fig. 24 dargestellt ist. Die Zusatzvorrichtung kann dann leicht an Marknägel unterschiedlicher Durchmesser angepaßt werden, indem man die Laschen längs einer Längsnut 225a abschneidet.

In dem vom Körper 222 umschlossenen Raum ist eine Aufnehmerspulenanordnung 227 angeordnet, welche eine Aufnehmerspule 224 enthält, die beispielsweise aus einer einlagigen Wicklung mit etwa 60 bis 70 Windungen eines bandförmigen Leiters bestehen kann. In der Aufnehmerspule befindet sich ein biegsamer Magnetkern 226, der aus einem Bündel von Drahten aus einem hochpermeablen Magnetmaterial, wie Mumetall besteht. Die Aufnehmerspule 224 und der Magnetkern 226 sind mit einem biologisch verträglichen Kunststoff 229 umgeben oder in diesen eingebettet.

Die Elektrodenanordnung 236 besteht aus einer Reihe von heftklammerartig geformten Elektrodenstreifen 236a, 236b, . . . (Fig. 22), die an ihren Stirnseiten durch Punktschweißungen miteinander verbunden sind, wie durch den Punkt auf der Elektrode 236a in Fig. 21 angedeutet ist. Die Schenkel der Elektrodenstreifen 236a, 236b, . . . sind durch entsprechende Löcher im mittleren Teil des Körpers 222 hindurchgesteckt und an den Enden umgebogen,wie in Fig. 22 und 23 dargestellt ist, so daß eine feste und sichere Verbindung der Elektrodenanordnung 236a mit dem Körper 222 gewährleistet ist. Die Breite der Elektrodenstreifen entspricht im wesentlichen der Breite des Zwischenraumes zwischen den Längsrippen 230 (Fig. 21).

Zur Halterung der Aufnehmerspulenanordnung 227 im Körper 222 dienen kurze Stücke aus Kunststoffrohr 233, die durch die umgebogenen Enden von Elektrodenstreifen am Körper befestigt sind, wie Fig. 22 zeigt.

Ein erstes Wicklungsende 229a der Aufnehmerspule ist mit einem Ende der Elektrodenanordnung verbunden, z.B. verschweißt, wie es in Fig. 23 dargestellt ist.

Das andere Ende der Aufnehmerspule ist mit blanken Blechstreifen 228 aus gewebeverträglichem Metall verschweißt, die außen am Körper 222 befestigt sind und mit dem Marknagel 210 elektrisch Kontakt machen, wenn die Zusatzvorrichtung 220 in den Marknagel eingeschoben ist. Vorzugsweise ist auf beiden Seiten des Körpers je ein Streifen 228 vorgesehen.

Die Länge der Zusatzvorrichtung 220 kann beispielweise etwa 220 mm betragen. Der Abstand der Außenseiten der Längsrippen 230 kann etwa 3,5 mm betragen und ist so bemessen, daß der durch die Längsrippen 230 und die Elektrodenanordnung 236 gebildete Ansatz in den Längsschlitz des Marknagels 210 paßt und eine einwandfreie Isolation der Elektrodenanordnung 236 vom Marknagel gewährleistet ist. Der Durchmesser der umhüllten Aufnehmerspulenanordnung 227 kann etwa 4 mm betragen, der Außendurchmesser der Aufnehmerspule ist nur geringfügig kleiner.

Die oben beschriebenen Ausführungsbeispiele lassen sich in der verschiedensten Weise abwandeln ohne den Rahmen der Erfindung zu überschreiten. Merkmale, die bei bestimmten Ausführungsformen erwähnt wurden, können gegebenenfalls auch bein anderen Ausführungsformen Anwendung finden, ohne daß dies besonders betont ist.

Zur Umwandlung eines konventionellen Osteosyntheseimplantats, wie einer Knochenplatte oder eines Mark- bzw. Küntscher-Nagels wird also eine Zusatzvorrichtung geschaffen, die eine Aufnehmerspule mit Elektrodenanschlüssen enthält. Im Falle einer Knochenplatte ist die Zusatzvorrichtung mit Mitteln zur Befestigung an der Platte versehen. Die Elektrodenanschlüsse können mit isoliert in die Platte eingesetzten Knochenschrauben verbunden werden welche dann als Gewebeelektroden dienen. Wahlweise kann auch ein Elektrodenanschluß mit der Platte selbst verbunden werden, die dann als Gewebeelektrode wirkt. Im Falle eines Marknagels ist die Zusatzvorrichtung so ausgebildet, daß sie in den Marknagel einschiebbar ist. Die Zusatzvorrichtung enthält ebenfalls eine Aufnehmerspule mit zwei Elektrodenanschlüssen, von denen der eine mit einer Gewebeelektrode verbunden ist, die sich bei applizierter Zusatzvorrichtung im Längsschlitz des Nagels befindet. Der andere Elektrodenanschluß kann mit einer zweiten Gewebeelektrode verbunden sein, die wie die erste Gewebeelektrode sich ebenfalls im Längsschlitz befindet und von der ersten Gewebeelektrode getrennt ist, alternativ kann auch ein Anschluß an den Marknagel vorgesehen sein, der dann als Gewebeelektrode wirkt und aus elektrischleitendem Material bestehen oder ein solches an geeigneter Stelle enthalten muß.

## Patentansprüche

1. Zusatzvorrichtung (72) zum Umwandeln eines konventionellen Osteosyntheseimplantats· in Form einer Osteosyntheseplatte (70) in eine Elektro-Osteosyntheseplatte, die mit einer Aufnehmerspule (24a) und mindestens zwei Gewebeelektroden versehen ist, die mit Anschlüssen (76, 78) der Aufnehmerspule (24a) elektrisch verbunden sind, dadurch gekennzeichnet, daß die Aufnehmerspule (24a) in einem Körper (74, 74a) angeordnet ist, der die als Halterungsteile dienenden Anschlüsse (76, 78) zum Anklemmen an die Osteosyntheseplatte (70) aufweist, daß einer der Anschlüsse (76) so angeordnet und ausgebildet ist, daß er elektrischen Kontakt mit einer elektrisch leitenden, als Gewebeelektrode dienenden Oberfläche der Osteosyntheseplatte (70) macht, wenn die Zusatzvorrichtung (72) an der Osteosyntheseplatte (70) angebracht ist, und daß der zweite Anschluß (78) in an sich bekannter Weise mit einer Anschlußvorrichtung (80) zum elektrischen und mechanischen Verbinden mit einer isolierten Knochenschraube, die die zweite Gewebeelektrode bildet, ausgebildet ist.

2. Zusatzvorrichtung nach Anspruch 1, dadurch gekennzeichnet, daß der Körper (74, 74a) in an sich bekannter Weise länglich geformt und seitlich an die Osteosyntheseplatte (70) anlegbar ist, daß einige der vor-stehenden als Halterungsteile dienenden An-schlüsse (76) mit der elektrisch leitenden Oberfläche der Osteosyntheseplatte (70) elektrisch Kontakt machen und so geformt sind, daß sie unter den Rand der an einem Knochen angebrachten Osteosyntheseplatte (70) geschoben werden können, und daß die andere als vorstehend Halterungsteile dienenden Anschlüsse (78), die auf der entgegengesetzten Seite der Osteosyntheseplatte (70) angeordnet sind, jeweils eine der Anschlußvorrichtungen (80) für eine Knochenschraube tragen.

3. Zusatzvorrichtung nach Anspruch 2, gekennzeichnet durch zwei solcher Körper (74a, 74b), die in an sich bekannter Weise länglich geformt, seitlich an die Osteosyntheseplatte anlegbar und durch federnde Verbindungselemente (82) verbunden sind.

4. Zusatzvorrichtung nach Anspruch 2 oder 3, dadurch gekennzeichnet, daß die die Elektrodenanschlüsse tragenden Halterungsteile (78') federnd verlängerbar ausgebildet sind.

5. Zusatzvorrichtung (20, 220) zum Umwandeln eines einen Längsschlitz (12) aufweisenden hohlen Marknagels (10, 120, 210) in einen Elektromarknagel, der eine Aufnehmerspule (24, 118, 224) mit mindestens zwei Anschlüssen und mindestens zwei mit jeweils einem der Anschlüsse verbundene Gewebeelektroden aufweist, dadurch gekennzeichnet, daß die Aufnehmerspule (24, 118, 224) und mindestens eine der Gewebeelektroden (36, 36a, 36 ... 118a, 236) mit Isoliermaterial (22; 110, 122; 222) zu einem länglichen Gebilde vereinigt sind das in die Höhlung des Marknagels (10, 120, 210) paßt und in diese einführbar ist, daß das längliche Gebilde einen durch den Längsschlitz (12) des marknagels (10, 120, 210) reichenden Vorsprung (32, 114, 230) aufweist, auf dem die besagte, mit einem Anschluß verbundene Gewebeelektrode (36; 36a, 36b ... 118a; 236) bezüglich des Mark-nagels isoliert angeordnet ist, und daß der zweite Anschluß entweder in Form einer Elektrode (28, 124, 228) derart an dem länglichen Gebilde angeordnet ist, daß er mit dem aus Metall bestehenden Marknagel elektrisch Kontakt macht, wenn die Zusatzvorrichtung in den Marknagel eingeführt ist, oder mit einer zweiten, auf dem Vorsprung angeordneten Gewebeelektrode verbunden ist.

6. Zusatzvorrichtung nach Anspruch 5, dadurch gekennzeichnet, daß der zweite Anschluß (28) der Aufnehmerspule (24) als federartige, vom länglichen Gebilde bogenförmig nach außen gewolbte Elektrode (28) ausgebildet ist.

7. Zusatzvorrichtung nach Anspruch 5 oder 6 mit mehreren Gewebeelektroden (36a bis 36d), dadurch gekennzeichnet, daß das längliche Gebilde mehrere Ansätze (30a bis 30d) mit je einer Gewebeelektrode (36a bis 36d) aufweist, die mit unterschiedlichen Wicklungsanschlüssen der Aufnehmerspule (24) verbunden sind, und daß das längliche Gebilde zwischen jeweils zwei Elektroden durchtrennbar ist.

8. Zusatzvorrichtung nach Anspruch 5 oder 6, dadurch gekennzeichnet, daß mindestens ein Teil (118a, 118a') mindestens einer Windung der Wicklung der Aufnehmerspule (118, 118') als Gewebeelektrode ausgebildet ist.

9. Zusatzvorrichtung nach Anspruch 5 oder 6, dadurch gekennzeichnet, daß das Isoliermaterial einen Körper (222) mit näherungsweise hufeisenförmigem Querschnitt bildet, der die Aufnehmerspule (224) mit Abstand umschl-ließt und außen in der Mitte mit einer in seiner Längsrichtung verlaufenden Elektrodenanordnung (236) versehen ist, welche durch Isoliermaterial (230) gegen den Marknagel (210) isoliert ist.

10. Zusatzvorrichtung nach Anspruch 9, dadurch gekennzeichnet, daß die Elektrodenanordnung aus mehreren, mit ihren Stirnseiten miteinander verbundenen und am Körper befestigten klammerartigen Elektrodenteilen (236a, 236b) besteht.

11. Zusatzvorrichtung nach Anspruch 9 oder 10, dadurch gekennzeichnet, daß an den Seitenrändern des Körpers (222) Ausschnitte (223) vorgesehen sind, die die Biegsamkeit des Körpers erhöhen.

12. Zusatzvorrichtung nach einem der Ansprüche 5 bis 11, dadurch gekennzeichnet, daß in der Aufnehmerspule (118, 224) ein ferromagnetischer Kern (112, 226), der aus einem Bündel biegsamer Drähte aus Magnetmaterial besteht, angeordnet ist.

13. Zusatzvorrichtung nach einem der vorhergehenden Ansprüche, gekennzeichnet durch mindestens eine Gewebeelektrode, die mit einer halbleitenden oder isolierenden Schicht überzogen ist.

**Claims**

1. Attachment (72) for converting a conventional osteosynthesis implant in form of an osteosynthesis plate (70) into an electrified ostesynthesis plate provided with a pick-up coil (24a) and at least two tissue electrodes, said electrodes being electrically coupled to terminals (76, 78) of said pick-up coil (24a) characterized in that said pick-up coil (24a) is positioned within a body (74, 74a) having said terminals (76, 78) serving as holding members for clamping to said osteosynthesis plate (70); that one of said terminals (76) is positioned and constructed so that it makes electrical contact with an electrically conductive surface of said osteosynthesis plate (70), when said attachement is attached to said osteosynthesis plate (70), said electrically conductive surface serving as tissue electrode; and that the second terminal (78) is provided, as known per se, with a connecting device (80) for electrical and mechanical connection to an insulated bone screw which forms the second tissue electrode.

2. The attachment in accordance with claim 1, characterized in that said body (74, 74a), as known per se, has an elongated form and is adapted to be positioned to the side of the osteosynthesis plate (70), that some of said protruding terminals (76) serving as holding members, make electrical contact with said electrically conductive surface of said osteosynthesis plate (70) and are formed such that they can be pushed under the edge of an osteosynthesis plate (70) attached to a bone, and that the other holding members serving as protruding terminals (78), which are positioned on the opposite side of the osteosynthesis plate (70), are each carrying a connecting device (80) for a bone screw.

3. The attachment in accordance with claim 2 characterized by a pair of such bodies (74a, 74b) which are of elongated form, as known per se, adapted to be positioned to the sides of said osteosynthesis plate, and are connected by resilient connecting elements (82).

4. The attachment as claimed in claims 2 or 3, characterized in that said holding members (78') which carry electrode terminals are resiliently stretchable.

5. Attachment (20, 220) for converting a hollow marrow nail (10, 120, 210) having a longitudinal slot (12) into an electrified marrow nail comprising a pick-up coil (24, 118, 224) having at least two terminals, and at least two tissue electrodes each connected to one of said terminals characterized in that said pickup coil (24, 118, 224) and at least one of said tissue electrodes (36, 36a, 36b ... 118a, 236) are combined with insulating material (22; 110; 122; 222) to an elongated structure fitting and insertable in the hollow of said marrow nail (10, 120, 210); that said elongated structure has a projection (32, 114, 230) extending through the longitudinal slot (12) of said marrow nail (10, 120, 210) said tissue electrode (36; 36a, 36b ...; 118a; 236) which is connected to a terminal, being positioned on said projection in insulated relationship with respect to said marrow nail, and that said second terminal either is positioned on said elongated structure in form of an electrode (28, 124, 228) such that it makes electrical contact with said marrow nail, when said attachement is inserted into said marrow nail which is made of metal, or is connected with a second tissue electrode positioned on said projection.

6. The attachment according to claim 5 characterised in that said second terminal (28) of said pick-up coil (24) is formed as a springlike electrode (28) curving outwardly from said elongated structure in an arch-like manner.

7. The attachment according to claims 5 or 6 having a plurality of tissue electrodes (36a to 36d) characterized in that said elongated structure has a plurality of projections (30a to 30d) with one tissue electrode (36a to 36d) each, said tissue electrodes being connected to different winding terminals of said pick-up coil (24); and that said elongated structure is adapted to be severed between adjacent electrodes.

8. The attachment according to claims 5 or 6 characterized in that at least a portion (118a, 118a') of at least one turn of the winding of said pick-up coil (118, 118') is formed as tissue electrode.

9. The attachment according to claims 5 or 6 characterized in that said insulating material forms a body (222) of essentially horseshoeshaped cross section enclosing said pick-up coil (24) in spaced relationship and being provided at the middle of its outer side with a longitudinally extending electrode arrangement (236) which is insulated by insulating material (230) against said marrow nail (210).

10. The attachment according to claim 9 characterised in that said electrode arrangement comprises a plurality of bracket-like electrode portions (236a, 236b) connected with their front sides.

11. The attachment in accordance with claims 9 or 10 characterized in that cut-out portions (223) are provided at the side edges of said body (222) to improve the flexibility of said body.

12. The attachment according to any of claims 5 to 11, characterized in that a ferromagnetic core (112, 226) consisting of a bundle of flexible wires of magnet material is positioned in the pick-up coil (118, 224).

13. The attachment as claimed in any of the preceding claims characterized by at least one tissue electrode which is coated with a semiconductive or insulating layer.

**Revendications**

1. Dispositif additionnel (72) pour la transformation d'un implant d'ostéosynthèse conventionnel, constitué par une plaque d'ostéosynthèse (70), en une plaque d'électro-ostéosynthèse comprenant une bobine réceptrice (24a) et au moins deux électrodes de tissu reliées électriquement à des connexions (76, 78) de la bobine réceptrice (24a), ledit dispositif étant caractérisé en ce que la bobine réceptrice (24a) est logée dans un corps (74, 74a) qui comporte des connexions (76, 78) servant de pièces de fixation pour le serrage sur la plaque d'ostéosynthèse (70); une des connexions est réalisée et disposée de façon à établir un contact électrique avec une surface de la plaque d'ostéosynthése (70), conductrice et servant d'électrode de tissu, quand le dispositif additionnel (72) est placé sur ladite plaque (70); et la seconde connexion (78) est réalisée de façon connue avec une pièce de connexion (80) pour l'établissement d'une liaison électrique et mécanique avec une vis isolée, constituant la seconde électrode de tissu.

2. Dispositif additionnel selon revendication 1, caractérisé en ce que le corps (74, 74a), de forme allongée connue, s'applique latéralement sur la plaque d'ostéosynthèse (70); certaines des connexions (76) en saillie et formant des pièces de fixation établissent un contact électrique avec la surface conductrice de la plaque d'ostéosynthèse (70) et sont formées de façon à pouvoir s'insérer sous le bord de la plaque d'ostéosynthèse (70) placée sur un os; et les autres connexions (78) en saillie, formant des pièces de fixation et disposées sur le côté opposé de la plaque d'ostéosynthèse (70), portent chacune une pièce de connexion (80) pour une vis.

3. Dispositif additionnel selon revendication 2, caractérisé par deux corps (74a, 74b), de forme allongée connue, s'appliquant latéralement sur la plaque d'ostéosynthèse et réunis par des éléments élastiques de liaison (82).

4. Dispositif additionnel selon une des revendications 2 et 3, caractérisé en ce que les pièces de fixation (78') portant les connexions d'electrode sont allongeables élastiquement.

5. Dispositif additionnel (20, 220) pour la transformation d'une broche centromédullaire (10, 120, 210), creuse et comportant une fente longitudinale (12), en une électro-broche, comportant une bobine réceptrice (24, 118, 224) avec au moins deux bornes et au moins deux electrodes de tissu reliées chacune à une desdites bornes, ledit dispositif additionnel étant caractérisé en ce que la bobine réceptrice (24, 118, 224) et au moins une des électrodes de tissu (36, 36a, 36b ... 118a, 236) sont réunies avec un matériau isolant (22; 110, 122; 222) pour former une structure allongée qui s'introduit et s'ajuste dans la cavité de la broche centromédullaire (10, 120, 210); la structure allongée présente une saillie (32, 114, 230) qui traverse la fente longitudinale (12) de la broche centromédullaire (10, 120, 210) et sur laquelle l'électrode de tissu (36; 36a; 36b ... 118a;236) reliée à une connexion est isolée par rapport à la broche centromédullaire; et la seconde connexion est soit réalisée sous forme d'une électrode (28, 124, 228) disposée sur la structure allongée de façon à établir un contact électrique avec la broche centromédullaire métallique, lors de l'introduction du dispositif additionnel dans ladite broche, soit reliée à une seconde

electrode de tissu, disposée sur la saillie.

6. Dispositif additionnel selon revendication 5, caractérisé en ce que la seconde connexion (28) de la bobine réceptrice (24) est réalisée sous forme dune électrode (28) élastique, bombée vers l'extérieur sur la structure allongée.

7. Dispositif additionnel selon une des revendications 5 et 6, comportant plusiers électrodes de tissu (36a à 36d) et caractérisé en ce que la structure allongée comporte plusieurs saillies (30a à 30d), munies chacune d'une électrode de tissu (36a à 36d) et reliées à des bornes différentes de la bobine recéptrice (24); et la structure allongée peut être sectionnée entre deux électrodes.

8. Dispositif additionnel selon une des revendications 5 et 6, caractérisé en ce qu'une partie au moins (118a, 118a') d'une spire au moins du bobinage de la bobine réceptrice (118, 118') est réalisée en électrode de tissu.

9. Dispositif additionnel selon une des revendications 5 et 6, caractérisé en ce que le matériau isolant forme un corps (222) de section sensiblement en fer à cheval, entourant à distance la bobine réceptrice (224) et muni extérieurement et au centre d'un groupe d'électrodes (236), longitudinal et isolé de la broche centromédullaire (210) par un matériau isolant (230).

10. Dispositif additionnel selon revendication 9, caractérisé en ce que le groupe d'électrodes est constitué par plusieurs pièces d'électrode (236a, 236b) en forme d'agrafe, fixées sur le corps et dont les faces frontales sont réunies.

11. Dispositif additionnel selon une des revendications 9 et 10, caractérisé en ce que les bords latéraux du corps (222) comportent des évidements (223) qui augmentent la soupless du corps.

12. Dispositif additionnel selon une quelconque des revendications 5 à 11, caractérisé en ce que la bobine réceptrice (118, 224) contient un noyau ferromagnétique (112, 226), constitué par un faisceau de fils souples d'un matériau magnétique.

13. Dispositif additionnel selon une quelconque des revendications 1 à 12 caractérisé par au moins une électrode de tissu, revêtue d'une couche semiconductrice ou isolante.

Fig. 1

Fig. 2

Fig. 3

Fig. 4

Fig. 5

Fig. 6

Fig. 7

1

Fig.8

Fig. 9

Fig.10

Fig.11

Fig.12

Fig.13

Fig.15

Fig.16

Fig.17

Fig.14

Fig.18

## Fig. 20

## Fig. 21

## Fig. 19

## Fig. 22

## Fig. 23

## Fig. 24

4